# EUROPÄISCHE PATENTSCHRIFT

(11) **EP 0 492 317 B1**
(45) Veröffentlichungstag und Bekanntmachung des Hinweises auf die Patenterteilung: **25.01.1995**
(21) Anmeldenummer: 91121416.1
(22) Anmeldetag: 13.12.1991
(51) Int. Cl.: A61F 5/01

(54) **Dynamische Redressionsschiene zur Therapie der Vorfussadduktion**
Dynamic redression splint for the therapy of the forefoot adduction
Atelle dynamique de redression pour la thérapie de l'adduction de l'avant-pied

(30) Priorität: 20.12.1990 DE 9017211 U; 18.06.1991 DE 9107481 U
(43) Veröffentlichungstag der Anmeldung: 01.07.1992
(73) Patentinhaber: Ostfolk, Rudi, 66903 Dittweiler (DE)
(72) Erfinder: Ostfolk, Rudi, 66903 Dittweiler (DE)
(74) Vertreter: Patentanwälte Möll und Bitterich

(56) Entgegenhaltungen:
- EP-A- 0 128 115
- EP-A- 0 130 915
- DE-A- 2 651 469
- DE-A- 3 543 642
- US-A- 4 632 096

## Beschreibung

Die Erfindung betrifft eine orthopädische Redressions-Schiene zur adjuvanten Therapie der angeborenen Vorfußadduktion oder der operierten Sichelfußdeformität gemäß dem Oberbegriff des Anspruchs 1.

Angeborene Sichelfuß- und Klumpfußdeformitäten zeichnen sich unter anderem durch eine mediale Abweichung des Vorfußes aus. Der Klumpfuß zeigt neben dem Fersenhochstand noch eine Supinationsfehlstellung des Vorfußes. Je nach Schwere der Deformität gelingt es, den fuß entweder allein mit Hilfe von orthopädischen Redressions-Schienen oder mit einer unterstützenden Operation in die Normalstellung zu bringen. Die bekannten Schienen bestehen aus einem Oberschenkelschaft und einem Unterschenkelschaft mit angeformter fußschale. Wesentliches Teil einer solchen Schiene ist ein spezielles Vorfußteil, welches den Vorfuß des Patienten schalenartig umfaßt und in die Korrekturstellung zwingt. Mit fortschreitendem Therapieerfolg muß die orthopädische Schiene immer wieder nachgearbeitet und angepaßt werden. Dasselbe gilt, wenn der Patient während der Therapie wächst.

Zusätzlich führen die Patienten eine aktive Gymnastik mit Anleitung und Hilfestellung durch einen Therapeuten durch. Diese Gymnastik besteht im wesentlichen darin, daß der deformierte Fuß immer wieder in die Normalstellung und insbesondere darüber hinaus in eine überkorrigierte Stellung gebracht wird. Der Therapieerfolg dieser dynamischen Gymnastik ist anerkanntermaßen besser als der der statischen Schienen. Aus zeitlichen und finanziellen Gründen ist eine solche therapeutische Gymnastik jedoch nur in begrenztem Umfang durchführbar.

Hier setzt die vorliegende Erfindung ein. Sie hat sich die Aufgabe gestellt, eine orthopädische Schiene der eingangs genannten Art dahingehend weiterzuentwickeln, daß eine laterale und pronierende Redression dosiert und korrigierend möglich wird.

Diese Aufgabe wird gelöst durch eine gattungsgemäße orthopädische Schiene mit den Merkmalen gemäß Kennzeichen des Anspruchs 1.

Damit ergeben sich die Vorteile, daß die Schiene nicht mehr nur statisch, sondern dynamisch korrigierend wirkt, wobei die entsprechenden Kräfte vom Patienten selbst aufgebracht und dosiert werden. Dies geschieht selbst bei Säuglingen durch Ausnutzung des reflektorischen Streckzwanges der unteren Extremitäten. Die Schiene selbst ist leicht. Durch definierte Auslegung der Koppelmechanik können etwaige überdehnungen sicher verhindert werden. Dank der dynamischen Korrekturwirkung ist ein beschleunigter Heilungsverlauf zu erwarten.

Gemäß einer bevorzugten Ausgestaltung der Erfindung kann die Koppelmechanik als Kurbeltrieb, als Gummizug oder auch als Seilzug realisiert werden. Ein Gummizug hätte dabei den Vorteil, daß Fehleinstellungen der Übersetzung elastisch ausgeglichen werden.

Als besonders geeignet hat sich ein Bowdenzug herausgestellt, dessen Hülle am Unterschenkelschaft und dessen beide Enden am Oberschenkelschaft bzw. am Vorfußteil befestigt sind. Bowdenzüge sind leicht, betriebssicher und verletzungsarm.

Oberschenkelschaft und Unterschenkelschaft können entweder direkt mittels Niet- oder Schraubachsen oder aber unter Verwendung von speziellen Kniegelenkschienen miteinander verbunden sein. Um beim Wachsen des Patienten eine entsprechende Längenveränderung wenigstens in gewissen Grenzen zu ermöglichen, sind Gelenkschiene, Oberschenkelschaft und/oder Unterschenkelschaft mit entsprechenden Einrichtungen, insbesondere in Form von Bohrungen, ausgerüstet.

Um die erforderliche laterale und pronierende Redression zu ermöglichen, muß das Vorfußteil mit einer entsprechenden Mechanik mit der Rückfußschale verbunden sein. Hierzu ist an der Rückfußschale und insbesondere an einer an ihrer Unterseite befestigten Metallplatte ein erster Achszapfen starr und etwa parallel dazu ein zweiter Achszapfen schwenkbar befestigt. An bzw. unter dem Vorfußteil ist eine Querwinkelleiste befestigt. Diese Querwinkelleiste trägt eine Buchse, in der der zweite Achszapfen drehbar gehalten ist. Weiterhin ist in die Querwinkelleiste ein radialer Führungsschlitz eingearbeitet, durch den der erste Achszapfen durchgesteckt ist.

Um die Gelenkmechanik zu schützen und eine etwaige Verletzungsgefahr zu verringern, ist die Unterseite von Rückfußschale und Vorfußteil mit einem Schutzdeckel abgedeckt.

Zur Befestigung der orthopädischen Schiene am Patientenbein sind Bänder mit Klettverschluß vorgesehen, die ein einfaches Anlegen und Abnehmen sowie einen sicheren Halt ermöglichen.

Anhand der Zeichnung soll die Erfindung in Form eines Ausführungsbeispiels näher erläutert werden. Es zeigen
- Fig. 1: eine dynamische Redressionsschiene zur Therapie des Sichelfußes in Seitenansicht,
- Fig. 2: das Vorfußteil der orthopädischen Schiene in Frontsicht und
- Fig. **3**: die Konstruktion des Lagers zwischen Rückfußschale und Vorfußteil in Draufsicht.

Die dargestellte orthopädische Redressionsschiene umfaßt einen Oberschenkelschaft 1, einen Unterschenkelschaft 2 mit angeformter Rückfußschale 21 und ein Vorfußteil 3. Diese Teile bestehen aus einem thermoplastischen Kunststoff und werden in bekannter Weise paßgenau an Bein und fuß des Patienten angeformt. Die Befestigung der Teile am Patientenbein erfolgt mit Hilfe von Bändern 7 mit Klettverschluß.

Oberschenkelschaft 1 und Unterschenkelschaft 2 sind mittels Kniegelenkschienen 9 schwenkbar miteinander verbunden. Die Gelenkschiene 9 ist mit einer Mehrzahl von Bohrungen ausgerüstet, um beim Wachsen des Patienten eine Längenanpassung zu ermöglichen, ohne gleich eine neue Therapieschiene anfertigen zu müssen.

Statt die in der Zeichnung dargestellte Gelenkschiene 9 zu verwenden, können Oberschenkelschaft 1 und Unterschenkelschaft 2 auch direkt mittels Niet- bzw. Schraubachsen gelenkig miteinander verbunden werden.

Das Vorfußteil 3 ist an der Rückfußschale 21 so angekoppelt, daß es um die Fußlängsachse kippbar und in der fußsohlenebene schwenkbar ist. Dadurch wird die kombinierte laterale und pronierende Redression ermöglicht.

Zur Übertragung der aktiven Oberschenkelstreckung auf das Vorfußteil 3 ist eine geeignete Koppelmechanik vorgesehen. Diese besteht hier aus einem Bowdenzug 11, dessen Hülle 12 am Unterschenkelschaft 2 befestigt ist. Die beiden Enden 4 des Bowdenzugdrahtes 11 sind mit je einer Pelottenknopfschraube am Oberschenkelschaft 1 und am Vorfußteil 3 befestigt. Dabei ist es möglich, durch entsprechende Einstellung der Hebelverhältnisse die Bewegungswinkel des Vorfußteils 3 in Richtung auf eine dynamische Überkorrektur einzustellen, wobei der fortschreitende Heilungsverlauf berücksichtigt werden kann.

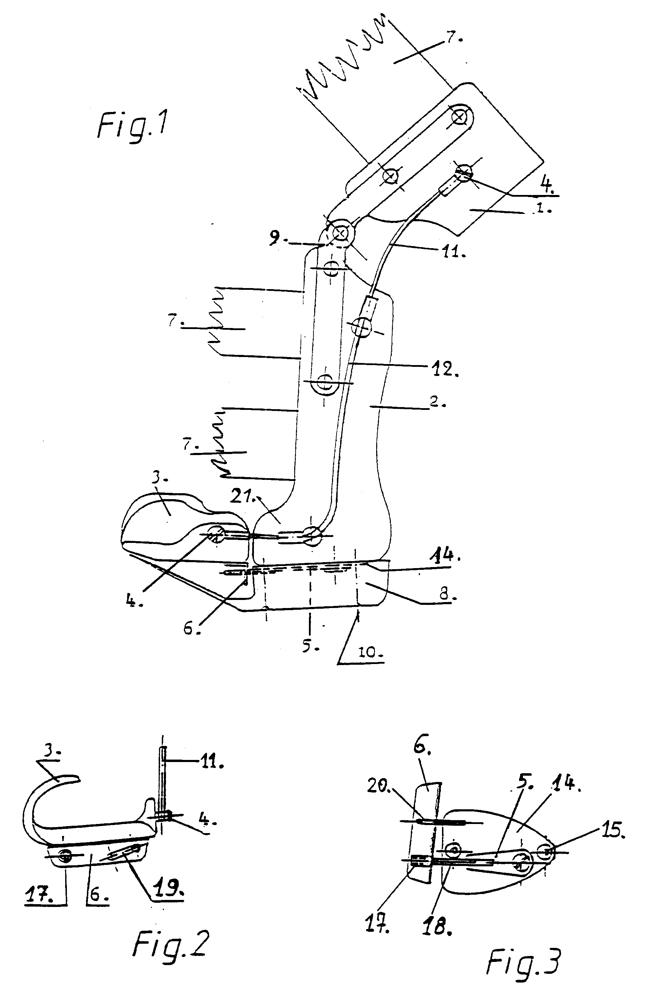

Die Fig. 2 und 3 zeigen eine Konstruktion, die die kombinierte pronierende und laterale Bewegung des Vorfußteils ermöglicht. Unter der Rückfußschale 21 ist eine Metallplatte 14 mit Hilfe von Schrauben 10, die durch Bohrungen 15 geführt sind, befestigt. An der Metallplatte 14 ist eine erste Achse 20 starr, eine zweite Achse 18 mittels eines Schwenkblechs 5 in der Fußsohlenebene schwenkbar befestigt.

Unter dem Vorfußteil 3 ist ein Winkelblech 6 montiert. Dieses besitzt ein radiales Langloch 19, durch das die erste Achse 20 gesteckt wird. Ferner ist am Winkelblech 6 eine Buchse 17 befestigt, in der die zweite Achse 18 drehbar gehalten ist.

Als Schutz vor Verletzungen und Beschädigungen ist die Unterseite von Rückfußschale 21 und Vorfußteil 3 durch eine Schutzkappe 8 abgedeckt.

Insgesamt bietet diese Schiene dem Patienten die Möglichkeit, auch ohne Therapeuten allein durch Eigenenergie eine körperlich steuerbare und dosierbare korrigierende passive Flexion mit kurzzeitiger dynamischer Überkorrektur der Fußstellung zu beliebigen Zeiten und an beliebigen Orten durchzuführen, wodurch ein schneller Therapieerfolg möglich wird.

## Patentansprüche

1. Orthopädische Redressions-Schiene zur adjuvanten Therapie der angeborenen Vorfußadduktion oder der operierten Sichelfußdeformität, mit einem Oberschenkelschaft (1), einem Unterschenkelschaft (2) mit angeformter Rückfußschale (21) und einem den Vorfuß in die Normalstellung zwingenden Vorfußteil (3), dadurch gekennzeichnet, daß Oberschenkelschaft (1) und Unterschenkelschaft (2) gelenkig miteinander verbunden sind, daß das Vorfußteil (3) an der Rückfußschale (21) um die Fußlängsachse kippbar und in der Fußsohlenebene schwenkbar angelenkt ist und daß eine Koppelmechanik vorgesehen ist, die beim Strecken des Kniegelenks das Schwenken und Kippen des Vorfußteils (3) bewirkt.

2. Schiene nach Anspruch 1, dadurch gekennzeichnet, daß die Koppelmechanik als Kurbeltrieb realisiert ist.

3. Schiene nach Anspruch 1, dadurch gekennzeichnet, daß die Koppelmechanik als Gummizug realisiert ist, dessen Enden (4) am Oberschenkelschaft (1) und am Vorfußteil (3) befestigt sind.

4. Schiene nach Anspruch 1, dadurch gekennzeichnet, daß die Koppelmechanik als Seilzug (11) realisiert ist, dessen Enden (4) am Oberschenkelschaft (1) und am Vorfußteil (3) befestigt sind.

5. Schiene nach Anspruch 1 oder 4, dadurch gekennzeichnet, daß die Koppelmechanik als Bowdenzug (11, 12) realisiert ist, dessen Hülle (12) am Unterschenkelschaft (2) befestigt ist.

6. Schiene nach einem der Ansprüche 1 bis 5, dadurch gekennzeichnet, daß zur gelenkigen Verbindung von Oberschenkelschaft (1) und Unterschenkelschaft (2) wenigstens eine Kniegelenkschiene (9) vorgesehen ist.

7. Schiene nach einem der Ansprüche 1 bis 6, dadurch gekennzeichnet, daß Oberschenkelschaft (1) und Unterschenkelschaft (2) mittels Niet- oder Schraubachsen gelenkig miteinander verbunden sind.

8. Schiene nach Anspruch 6 oder 7, dadurch gekennzeichnet, daß Gelenkschiene (9), Oberschenkelschaft (1) und/oder Unterschenkelschaft (2) Einrichtungen, insbesondere in Form von Bohrungen, zur Längenveränderung besitzen.

9. Schiene nach einem der Ansprüche 1 bis 8, dadurch gekennzeichnet, daß zur gelenkigen Verbindung des Vorfußteils (3) mit der Rückfußschale (21) an der Rückfußschale (21) ein erster Achszapfen (20) starr und ein zweiter Achszapfen (18) schwenkbar befestigt sind, daß am Vorfußteil (3) eine Querwinkelleiste (6) befestigt ist, daß an der Querwinkelleiste (6) eine Buchse (17) befestigt ist, in der der zweite Achszapfen (18) drehbar gehalten ist, und daß in die Querwinkelleiste (6) ein radialer Führungsschlitz (19) eingearbeitet ist, durch den der erste Achszapfen (20) durchgesteckt ist.

10. Schiene nach einem der Ansprüche 1 bis 9, dadurch gekennzeichnet, daß erster und zweiter Achszapfen (20, 18) an einer Metallplatte (14) unter der Rückfußschale (21) befestigt sind.

11. Schiene nach einem der Ansprüche 1 bis 10, dadurch gekennzeichnet, daß die Unterseite der Rückfußschale (21) und des Vorfußteils (3) mit einem Schutzdeckel (8) abgedeckt ist und daß zur Befestigung von Oberschenkelschaft (1) und Unterschenkelschaft (2) Bänder (7) mit Klettverschluß vorgesehen sind.

## Claims

1. An orthopaedic redression splint for adjuvant therapy of congenital forefoot adduction or operated skew-foot deformity, having a thigh shaft (1), a lower leg shaft (2) with an integrally formed tarsal shell (21) and a forefoot part (3) constraining the forefoot into a normal position, characterised in that the thigh shaft (1) and the lower leg shaft (2) are connected to one another in an articulated manner, in that the forefoot part (3) is articulated to the tarsal shell (21) so as to be able tilt about the longitudinal axis of the foot and so as to be pivotable in the plane of the sole of the foot, and in that a coupling mechanism is provided which induces the pivoting and tilting of the forefoot part (3) when the knee joint is extended.

2. A splint according to Claim 1, characterised in that the coupling mechanism is in the form of a crank assembly.

3. A splint according to Claim 1, characterised in that the coupling mechanism is in the form of a rubber band, the ends (4) of which are fastened to the thigh shaft (1) and to the forefoot part (3).

4. A splint according to Claim 1, characterised in that the coupling mechanism is in the form of a cable control (11), the ends (4) of which are fastened to the thigh shaft (1) and to the forefoot part (3).

5. A splint according to Claim 1 or 4, characterised in that the coupling mechanism is in the form of a Bowden cable (11, 12), the sheath (12) of which is secured to the lower leg shaft (2).

6. A splint according to any one of Claims 1 to 5, characterised in that at least one knee joint bar (9) is provided for the articulated connection of the thigh shaft (1) and the lower leg shaft (2).

7. A splint according to any one of Claims 1 to 6, characterised in that the thigh shaft (1) and the lower leg shaft (2) are connected to one another in an articulated manner by means of rivet or screw spindles.

8. A splint according to Claim 6 or 7, characterised in that the joint bar (9), thigh shaft (1) and/or lower leg shaft (2) have means, in particular in the form of bores, for length variation.

9. A splint according to any one of Claims 1 to 8, characterised in that for the articulated connection of the forefoot part (3) to the tarsal shell (21) a first swivel pin (20) is mounted rigidly and a second swivel pin (18) is mounted pivotably on the tarsal shell (21), in that a transverse angle bar (6) is secured to the forefoot part (3), in that on the transverse angle bar (6) there is secured a bush (17) in which the second swivel pin (18) is rotatably mounted, and in that the transverse angle bar (6) incorporates a radial guide slot (19) through which the first swivel pin (20) is fitted.

10. A splint according to any one of Claims 1 to 9, characterised in that first and second swivel pins (20, 18) are mounted on a metal plate (14) under the tarsal shell (21).

11. A splint according to any one of Claims 1 to 10, characterised in that the underside of the tarsal shell (21) and of the forefoot part (3) is covered by a protective cover (8), and in that bands (7) with a hook-and-loop type fastening are provided for securing the thigh shaft (1) and the lower leg shaft (2).

## Revendications

1. Attelle de rééducation orthopédique pour la thérapie adjuvante de l'adduction congénitale de l'avant-pied, ou bien de la malformation opérée du pied bot, comportant une tige de cuisse (1), une tige de jambe (2) avec une coque d'arrière-pied (21) intégrée et une partie d'avant-pied (3), contraignant l'avant-pied dans la position normale, caractérisée en ce que la tige de cuisse (1) et la tige de jambe (2) sont reliées de façon articulée, en ce que la partie d'avant-pied (3) est reliée à la coque d'arrièrepied (21) de manière à pouvoir basculer autour de l'axe longitudinal du pied et pouvoir pivoter dans le plan de la semelle du pied, et en ce qu'est prévu un mécanisme de couplage, qui provoque le pivotement et le basculement de la partie d'avant-pied (3) lors de l'étirement de l'articulation du genou.

2. Attelle selon la revendication 1, caractérisée en ce que le mécanisme de couplage est réalisé sous forme de mécanisme à bielle-manivelle.

3. Attelle selon la revendication 1, caractérisée en ce que le mécanisme de couplage est réalisé sous forme de tendeurs en caoutchouc, dont les extrémités (4) sont fixées sur la partie de cuisse (1) et sur la partie d'avant-pied (3).

4. Attelle selon la revendication 1, caractérisée en ce que le mécanisme de couplage est réalisé sous forme de câble de traction (11) dont les extrémités (4) sont fixées sur la partie de cuisse (1) et sur la partie d'avant-pied (3).

5. Attelle selon la revendication 1 ou 4, caractérisée en ce que le mécanisme de couplage est réalisé sous forme de câble de Bowden (11, 12), dont la gaine (12) est fixée sur la tige de jambe (2).

6. Attelle selon l'une des revendications 1 à 5, caractérisée en ce qu'au moins un attelle d'articulation de genou (9) est prévue pour assurer la liaison articulée entre la tige de cuisse (1) et la tige de jambe (2).

7. Attelle selon l'une des revendications 1 à 6, caractérisée en ce que la tige de cuisse (1) et la tige de jambe (2) sont reliées ensemble de façon articulée, ou au moyen d'axes rivetés ou vissés.

8. Attelle selon la revendication 6 ou 7, caractérisée en ce que l'attelle articulée (9) , la tige de cuisse (1) et/ou la tige de jambe (2) comportent des agencements, en particulier se présentant sous la forme de perçages, destinés à permettre une variation de longueur.

9. Attelle selon l'une des revendications 1 à 8, caractérisée en ce que, pour permettre une liaison articulée de la partie d'avant-pied (3) avec la coque d'arrière-pied (21), sur la coque d'arrière-pied (21), sont prévus un premier tourillon d'axe (20) monté rigide et un deuxième tourillon d'axe (18) monté pivotant, en ce que, sur la partie d'avant-pied (3) , est fixée une bande transversale en cornière (6) , en ce que sur la bande transversale en cornière (6) est fixée une douille (17), dans laquelle le deuxième tourillon d'axe (18) est maintenu tout en pouvant tourner, et en ce que dans la bande transversale en cornière (6) est ménagée une fente de guidage (19) radiale, à travers laquelle passe le premier tourillon d'axe (20).

10. Attelle selon l'une des revendications 1 à 9, caractérisée en ce que le premier et le deuxième tourillons d'axe (20, 18) sont fixés sur une plaque métallique (14) située sous la coque d'arrière-pied (21).

11. Attelle selon l'une des revendications 1 à 10, caractérisée en ce que les faces inférieures de la coque d'arrière-pied (21) et de la partie d'avant-pied (3) sont recouvertes par un couvercle de protection (8), et en ce que des bandes (7) avec une fermeture à accrochage sont prévues pour assurer la fixation de la tige de cuisse (1) et de la tige de jambe (2).
